# EUROPÄISCHE PATENTSCHRIFT

(11) **EP 3 217 185 B1**
(45) Veröffentlichungstag und Bekanntmachung des Hinweises auf die Patenterteilung: **08.08.2018**
(21) Anmeldenummer: 17153085.0
(22) Anmeldetag: 25.01.2017
(51) Int. Cl.: G01R 33/36, G01R 33/422, H01F 7/18

(54) **SCHALTUNGSANORDNUNG ZUR STROMVERSORGUNG EINER MAGNETRESONANZBILDGEBUNGSANLAGE**
CIRCUIT ASSEMBLY FOR THE POWER SUPPLY OF A MAGNETIC RESONANCE IMAGING INSTALLATION
CIRCUITERIE D'ALIMENTATION ÉLECTRIQUE D'UN ÉQUIPEMENT D'IMAGERIE PAR RÉSONANCE MAGNÉTIQUE

(30) Priorität: 09.03.2016 DE 102016203817
(43) Veröffentlichungstag der Anmeldung: 13.09.2017
(73) Patentinhaber: Siemens Healthcare GmbH, 91052 Erlangen (DE)
(72) Erfinder: Model, Volker, 90766 Fürth (DE)

(56) Entgegenhaltungen:
- EP-A1- 2 184 615
- DE-A1-102008 038 989
- JP-A- 2002 224 079

## Beschreibung

### Gebiet der Erfindung

Die Erfindung betrifft eine Magnetresonanzbildgebungsanlage mit einer Stromversorgung sowie ein Verfahren zum Betrieb einer Magnetresonanzbildgebungsanlage.

### Hintergrund der Erfindung

Die Magnetresonanzbildgebung (Magnetresonanztomographie MRT, kurz auch MR; auch Kernspintomographie genannt; Englisch: MRI für Magnetic Resonance Imaging) ist ein bildgebendes Verfahren, das vor allem bei der medizinischen Diagnostik zur Darstellung von Struktur und Funktion von Gewebe und Organen im menschlichen Körper eingesetzt wird. Es basiert auf den physikalischen Prinzipien der Kernspinresonanz und wird daher mitunter auch als Kernspintomographie bezeichnet. Allgemeine Details finden sich beispielweise in Wikipedia unter http://de.wikipedia.org/wiki/Magnetresonanztomographie.

Mit der Magnetresonanzbildgebung können Schnittbilder des menschlichen (oder tierischen) Körpers erzeugt werden, die eine Beurteilung der Organe und vieler krankhafter Organveränderungen erlauben. Die Magnetresonanztomographie basiert auf starken Magnetfeldern sowie elektromagnetischen Wechselfeldern im Hochfrequenzbereich, mit denen Atomkerne (meistens die Protonen des Wasserstoffs) resonant angeregt werden, wobei in einem Empfängerstromkreis elektrische Signale induziert werden. Bei der Magnetresonanzbildgebung werden weder Röntgenstrahlung noch eine andere ionisierende Strahlung erzeugt oder genutzt. Eine wesentliche Grundlage für den Bildkontrast bilden die unterschiedlichen Relaxationszeiten verschiedener Gewebearten. Daneben trägt auch der unterschiedliche Gehalt an Wasserstoffatomen in verschiedenen Geweben (z. B. Muskel, Knochen) zum Bildkontrast bei.

Um anhand der Magnetresonanzbildgebung ein Bild zu gewinnen, d.h. eine Magnetresonanzaufnahme eines Untersuchungsobjekts zu erzeugen, wird zunächst der Körper bzw. das zu untersuchende Körperteil des Patienten einem möglichst homogenen statischen Grundmagnetfeld ausgesetzt, das von einem Grundfeldmagneten des Magnetsystems der Magnetresonanzbildgebungsanlage erzeugt wird. Diesem Grundmagnetfeld werden während der Aufnahme der Magnetresonanzbilder schnell geschaltete Gradientenfelder zur Ortskodierung überlagert, die von den Gradientenspulen des Magnetsystems erzeugt werden. Außerdem werden mit einer Hochfrequenzantenne der Magnetresonanzbildgebungsanlage Hochfrequenzpulse einer definierten Feldstärke in das Untersuchungsvolumen eingestrahlt. Dazu weist die Magnetresonanzbildgebungsanlage in der Regel eine fest eingebaute Hochfrequenzantenne, die sogenannte Ganzkörperspule, auf. Mittels dieser Hochfrequenzpulse werden die Atome im Untersuchungsobjekt derart angeregt, dass sie um einen sogenannten "Anregungsflipwinkel" aus ihrer Gleichgewichtslage, die parallel zu dem Grundmagnetfeld verläuft, ausgelenkt werden. Die beim "Zurücklenken" erzeugten Magnetresonanzsignale werden von mindestens einer nicht ortsfesten Lokalspule erfasst und einer weiteren Verarbeitung zugeführt. Die Lokalspule ist dabei möglichst nahe an dem Patienten angeordnet, z.B. auf dem Patienten abgelegt.

Die Grundfeldmagnete sind in der Regel supraleitende Magnete, die eine regelbare Stromversorgung benötigen, mit der die Energie zum Aufbau des magnetischen Feldes zur Verfügung gestellt wird. Elektronische und elektrische Komponenten, die am Grundfeldmagneten der Magnetresonanzbildgebungsanlage verbaut sind, benötigen ebenfalls eine Stromversorgung. Die Verwendung von Schaltwandlern für eine energieeffiziente und verlustleistungsarme Stromversorgung gestaltet sich schwierig, da Vielfache der Schaltfrequenzen und deren Mischprodukte Störungen im MR-Signal verursachen können.

Aus der Praxis bekannt ist, die Stromversorgungen für die supraleitenden Magnete für einen Serviceeinsatz (Ramp-up, Ramp-down) als Service-Tool mitzuführen. Dies verursacht einen logistischen Aufwand und bedarf einer gründlichen Vorplanung. Ein sofortiger oder sogar spontaner Einsatz ist nicht möglich.

Eine weitere Möglichkeit zur Stromversorgung besteht darin, eine feste Installation vorzusehen, die ähnlich der mobilen Variante außerhalb des Wirkungsbereichs des magnetischen Feldes sowie außerhalb der für Magnetresonanzbildgebungsanlagen erforderlichen elektromagnetischen Schirmung (der sogenannten Hochfrequenzschirmungskabine bzw. HF-Kabine) aufgestellt sein muss.

Bei beiden beschriebenen Ausführungsformen muss der gesamte Magnetisierungsstrom (manchmal auch als "Magnetstrom" bezeichnet), der in aller Regel einige hundert Ampere beträgt, über lange Kabel und zum Teil durch Durchführungsfilter flie-ßen.

Stromversorgungen für die elektronischen und elektrischen Komponenten, die innerhalb der elektromagnetischen Schirmung betrieben werden, sind in der Regel außerhalb der Schirmkabine verbaut. Die Spannungszuführung erfolgt über eine Vielzahl von Leitungen, die jeweils Durchführungsfilter benötigen. In den Komponenten selbst werden meist Linearregler verwendet bzw. Schaltwandler mit entsprechender Filterung eingesetzt. Eine besondere Herausforderung stellt die Verwendung von ferrithaltigen induktiven Bauelementen oder (ferro-)magnetischen Bauteilen dar, da es hier zu Sättigungseffekten, einer Verformung des Grundmagnetfeldes und erheblichen Kraftwirkungen kommen kann.

Eine derartiges Stromversorgung gemäß dem Stand der Technik ist beispielhaft in der Offenlegungsschrift JP 2002 224089 A beschrieben.

Die Offenlegungsschrift DE 10 2008 038989 A1 offenbart eine Schaltungsanordnung zur Stromversorgung einer Magnetresonanzbildgebungsanlage. Sie weist erste Schaltungseinrichtung auf, die aus einer Netzspannung eine Zwischenkreis-Gleichspannung erzeugt und eine innerhalb der Hf-Abschirmungskabine angeordnete zweite Schaltungseinrichtung, die ausgebildet ist, aus der Zwischenkreis-Gleichspannung über piezoelektrische Gleichspannungswandler elektrische Verbraucher, z.B. Steuer- oder Regelungsanlagen für Gradienten und Hf-Einheiten, mit Spannung zu versorgen.

### Zusammenfassung der Erfindung

Es ist Aufgabe der Erfindung, ein eine Magnetresonanzbildgebungsanlage und ein Verfahren zum Betrieb einer Magnetresonanzbildgebungsanlage anzugeben, die eine einfache Speisung der supraleitenden Grundfeldmagneten ermöglichen.

Gemäß der Erfindung wird die gestellte Aufgabe mit der Magnetresonanzbildgebungsanlage und dem Verfahren zum Betrieb einer Magnetresonanzbildgebungsanlage der unabhängigen Patentansprüche gelöst. Vorteilhafte Weiterbildungen sind in den abhängigen Ansprüchen angegeben.

Erfindungsgemäß wird eine aus im Wesentlichen zwei Funktionsblöcken bestehende Schaltungsanordnung angegeben, wobei der erste Funktionsblock außerhalb der HF-Kabine angeordnet ist und eine Zwischenkreis-Gleichspannung erzeugt. Die Zwischenkreis-Gleichspannung wird über ein Kabel dem in der HF-Kabine befindlichen zweiten Funktionsblock mit Gleichspannungswandlern zugeführt, die den Magnetisierungsstrom für den Grundfeldmagneten liefern.

Ein Gleichspannungswandler, auch DC-DC-Wandler genannt, (Englisch: DC-DC Converter), bezeichnet eine elektrische Schaltung, die eine am Eingang zugeführte Gleichspannung in eine Gleichspannung mit höherem, niedrigerem oder invertiertem Spannungsniveau umwandelt. Die Umsetzung erfolgt mithilfe eines periodisch arbeitenden elektronischen Schalters und eines oder mehrerer Energiespeicher. Gleichspannungswandler zählen zu den selbstgeführten Stromrichtern. Im Bereich der elektrischen Energietechnik werden sie auch als Gleichstromsteller bezeichnet.

Die erfindungsgemäße Architektur setzt auf eine funktionale und lokale Trennung der einzelnen Bestandteile der Stromversorgung. Optional kann eine alternative Verwendung einzelner Funktionsblöcke vorgesehen werden. Die Auslegung der einzelnen Funktionsblöcke erfolgt gemäß dem Betriebszustand mit dem maximalen Leistungsbedarf, wobei davon ausgegangen wird, dass nicht alle Baugruppen in jedem Betriebszustand aktiv sein müssen.

Während des Aufladens des Grundfeldmagneten (Ramp-Up) wird die maximale Leistung für die Magnetisierungsstromversorgung benötigt. Andere elektrische und elektronische Komponenten werden beim Ramp-up nicht benötigt. Die Magnetisierungsstromversorgung ist daher fest installierter Bestandteil der Magnetresonanzbildgebungsanlage. Während die Magnetresonanzbildgebungsanlage in Betrieb ist, wird die Magnetisierungsstromversorgung nicht benötigt. Andere elektrische und elektronische Komponenten können optional Teile der Stromversorgung (z.B. Transformator, Gleichrichterschaltung, Verkabelung, Steuerung und Überwachung) nutzen.

Die Erfindung beansprucht eine Magnetresonanzbildgebungsanlage aufweisend eine Hochfrequenzschirmungskabine und mindestens einen Grundfeldmagneten. Die Anordnung weist ferner auf:
- eine außerhalb der Hochfrequenzschirmungskabine angeordnete erste Schaltungseinrichtung, die aus einer Netzspannung eine Zwischenkreis-Gleichspannung erzeugt, und
- eine innerhalb der Hochfrequenzschirmungskabine angeordnete, mit dem Grundfeldmagneten verbundene zweite Schaltungseinrichtung, die aus der Zwischenkreis-Gleichspannung einen Magnetisierungsstrom für den Grundfeldmagneten erzeugt.

Diese Architektur ermöglicht es, eine kosteneffiziente Lösung für eine integrierte (fest installierte) modulare Magnetisierungsstromversorgung zu realisieren. Die Leistungsübertragung zum Grundfeldmagneten mittels Zwischenkreis reduziert die Leitungsverluste. Kabel, Verbinder und Filter müssen für geringere Ströme ausgelegt werden. Die optionale, alternative Nutzung einzelner elektrischer und elektronischer Komponenten in den unterschiedlichen Betriebszuständen reduziert Schaltungs- und Kostenaufwände. Der modulare Aufbau ermöglicht eine flexible Anpassung der Systemstromversorgung an unterschiedliche Ausführungen einer Magnetresonanzbildgebungsanlage.

In einer Weiterbildung der Schaltungsanordnung kann die Netzspannung eine Dreiphasen-Wechselspannung sein.

In einer weiteren Ausführung weist die erste Schaltungseinrichtung in Reihe geschaltet einen Transformator und einen Dreiphasenwechselrichter auf.

In einer Weiterbildung kann der Dreiphasenwechselrichter als Sechspulsgleichrichter oder als Zwölfpulsgleichrichter ausgebildet sein.

In einer weiteren Ausbildung kann die zweite Schaltungseinrichtung eine Stromsenke aufweisen, die den Grundfeldmagneten bei Bedarf geregelt entlädt.

In einer weiteren Ausgestaltung weist die zweite Schaltungseinrichtung in Reihe geschaltet ein Eingangsfilter, mehrere parallel geschaltete erste Gleichspannungswandler, die den Magnetisierungsstrom liefern, und eine Strom-/Spannungsmesseinrichtung auf.

In einer Weiterbildung kann der erste Gleichspannungswandler als regelbare Stromquellen mit Spannungsbegrenzung ausgebildet sein.

In einer weiteren Ausgestaltung kann die zweite Schaltungseinrichtung eine Steuer- und Auswerteeinheit aufweisen, die die Stromsenke, die ersten Gleichspannungswandler und die Strom-/Spannungsmesseinrichtung steuert.

In einer weiteren Ausgestaltung weist die Anordnung ein die erste und die zweite Schaltungseinrichtung verbindendes Kabel mit mindestens einer Zuleitung und mit mindestens einer Rückleitung auf.

In einer Weiterbildung weist die zweite Schaltungseinrichtung einen von der Zwischenkreis-Gleichspannung gespeisten zweiten Gleichspannungswandler auf, der elektrische und elektronische Einheiten in der Hochfrequenzschirmungskabine mit Strom versorgt.

Außerdem beansprucht die Erfindung ein Verfahren zum Betrieb einer erfindungsgemäßen Magnetresonanzbildgebungsanlage, wobei zum Laden des Grundfeldmagneten die ersten Gleichspannungswandler eingeschaltet und der zweite Gleichspannungswandler ausgeschaltet werden.

In einer Weiterbildung des Verfahrens werden nach dem Aufladen des Grundfeldmagneten die ersten Gleichspannungswandler ausgeschaltet und der zweite Gleichspannungswandler eingeschaltet.

Weitere Besonderheiten und Vorteile der Erfindung werden aus den nachfolgenden Erläuterungen mehrerer Ausführungsbeispiele anhand von schematischen Zeichnungen ersichtlich.

### Es zeigen:

- Fig. 1:: ein Blockschaltbild einer Stromversorgung und
- Fig. 2:: ein Schaltbild einer Stromversorgung.

### Detaillierte Beschreibung mehrerer Ausführungsbeispiele

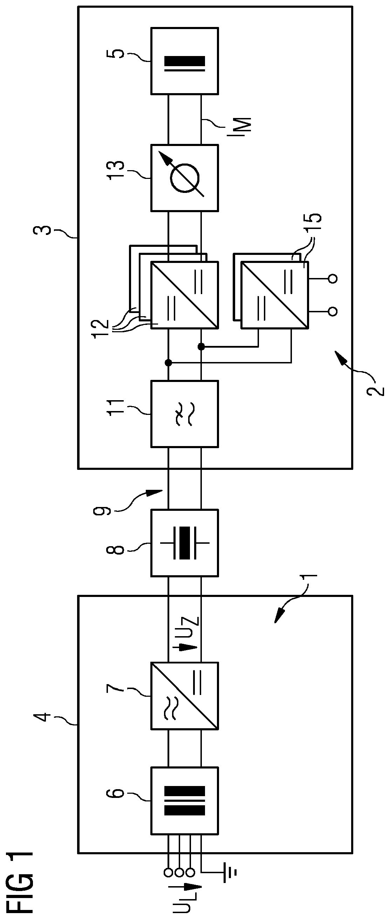

**Fig. 1** zeigt ein Blockschaltbild einer Stromversorgung zur Speisung eines Grundfeldmagneten 5 aufgeteilt in eine erste Schaltungseinrichtung 1 zur Erzeugung einer Zwischenkreis-Gleichspannung U_{Z} und in eine zweite Schaltungseinrichtung 2 zur eigentlichen Erzeugung des Magnetisierungsstroms I_{M}.

Eine Dreiphasenwechselstrom-Netzspannung U_{L} wird einem Transformator 6 zugeführt. Der niedertransformierte Wechselstrom liegt an einem Dreiphasen-Wechselrichter 7 an, der die Zwischenkreis-Gleichspannung U_{Z} erzeugt. Mit einem Durchführungsfilter 8 wird u.a. auch die Restwelligkeit verringert. Die Zwischenkreis-Gleichspannung U_{Z} hat einen Wert von maximal 60 V. Der Transformator 6 und der Dreiphasen-Wechselrichter 7 befinden sich in einem nicht-geschirmten Technikraum 4.

Der Dreiphasen-Wechselrichter (Gleichrichterschaltung) besteht im einfachsten Fall aus einem einzigen Gleichrichter, aus einem geschalteten Gleichrichter oder einem aktiven Gleichrichter mit PFC. Die Zwischenkreis-Gleichspannung U_{Z} ist dabei niedriger als der Spitzenwert der Netzspannung U_{L}, aber um ein Vielfaches höher als die Ausgangsspannung mit dem höchsten Leistungsbedarf. Das Spannungsverhältnis kann entweder mit dem Transformator 6 oder mit einem aktiven Gleichrichter eingestellt werden. Der Transformator 6 oder der Gleichrichter wird so ausgelegt, dass er die normativen Anforderungen an Netzteile in Hinblick auf Surge, Isolation, Leakage usw. erfüllt.

Über ein elektrisches Kabel 9 wird die Zwischenkreis-Gleichspannung U_{Z} einem Eingangsfilter 11 (im einfachsten Fall ein Kondensator) der zweiten Schaltungseinrichtung 2 zugeführt. Für die Stromversorgung des Grundfeldmagneten 5 wird somit nur ein einziges Kabel 9 mit Hin- und Rückleitung benötigt. Das Kabel 9 und das Eingangsfilter 11 sind entsprechend dem maximalen Leistungsbedarf ausgelegt. Die Strombelastbarkeit kann dabei gegenüber einer externen Magnetisierungsstromversorgung deutlich, nahezu im Verhältnis der Zwischenkreis-Gleichspannung U_{Z} zur Ausgangsspannung der Magnetisierungsstromversorgung, reduziert werden. Der Gleichspannungszwischenkreis reduziert ebenfalls Störungen und die durch die Lorenzkraft hervorgerufenen (wechselnden) Kraftwirkungen auf Bauteile und Kabel, besonders gegenüber einer Wechselstromzuführung.

Die so gefilterte Zwischenkreis-Gleichspannung U_{Z} wird nun in mehreren parallel geschalteten ersten Gleichspannungswandlern 12 auf ein niederes Spannungsniveau gebracht, um den erforderlichen Magnetisierungsstrom I_{M} in Höhe von etwa 400 A (bis max. 600 A) zum Laden (Ramp-up) des Grundfeldmagneten 5 bereitzustellen. Mit Hilfe der Strom-/Spannungsmesseinrichtung 13 werden der Magnetisierungsstrom I_{M} und die Ausgangsspannung überwacht. Mit Hilfe von zweiten Gleichspannungswandlern 15 können andere elektrische und elektronische Komponenten versorgt werden.

Das Eingangsfilter 11, die ersten Gleichspannungswandler 12, der zweite Gleichspannungswandler 15 und die Strom-/Spannungsmesseinrichtung befinden sich in der Hochfrequenzschirmungskabine 3 und sind Teil der zweiten Schaltungseinrichtung 2.

Die ersten Gleichspannungswandler 12 können auf Grund der relativ niedrigen Zwischenkreis-Gleichspannung U_{Z} mit einer hohen Taktfrequenz betrieben werden. Dabei können ferritfreie induktive Bauelemente eingesetzt werden, deren Funktion durch das Grundmagnetfeld nicht beeinflusst wird. Der zweite Gleichspannungswandler 15, der während des Betriebs der Magnetresonanzbildgebungsanlage aktiv ist, kann auch mit Frequenzen betrieben werden, die zum Frequenzplan der Magnetresonanzbildgebungsanlage passen und somit keine Störungen verursachen können.

Die Magnetisierungsstromversorgung wird durch mehrere parallel geschaltete Stromquellen mit Spannungsbegrenzung, die mit einem optimalen Puls-Pausen-Verhältnis betrieben werden können, oder mit ein oder mehreren parallelen hoch auflösenden Stromquellen ausgeführt. Aus dem Aufbau, dem Zu- und Wegschalten einzelner Module, ergibt sich die Funktion eines DAC. Diese Architektur kann auch auf andere Stromversorgungen für hohen Leistungsbedarf angewendet werden.

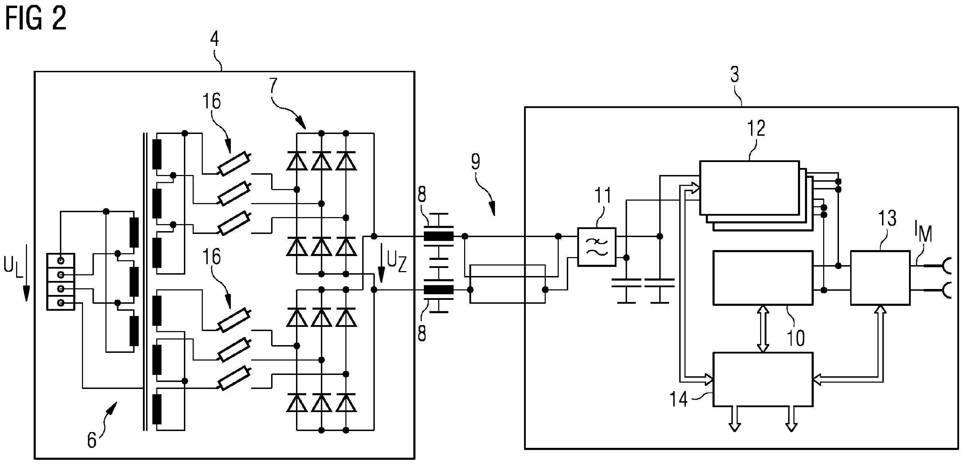

**Fig. 2** zeigt ein Schaltbild einer Stromversorgung für eine Magnetresonanzbildgebungsanlage, insbesondere die Stromversorgung für das Laden eines supraleitenden Grundfeldmagneten. Die Stromversorgung ist in zwei Funktionsblöcke unterteilt: eine erste Schaltungseinrichtung 1 im nicht geschirmten Technikraum 4 zur Erzeugung einer Zwischenkreis-Gleichspannung U_{Z} und eine zweite Schaltungseinrichtung 2 in der Hochfrequenzschirmungskabine 3 zur Erzeugung des Magnetisierungsstroms I_{M}. Die erste Schaltungseinrichtung 1 ist mit der zweiten Schaltungseinrichtung 2 über das Kabel 9 elektrisch verbunden. Zur Unterdrückung von Störungen werden vier verdrillte Adern benutzt, die paarweise kurzgeschlossen sind.

Die Dreiphasenwechselstrom-Netzspannung U_{L} wird einem Transformator 6 mit Eisenkern und sekundärseitig über thermomagnetische Schalter 16 (als Sicherung) einem Dreiphasen-Wechselrichter 7 zugeführt. Der Dreiphasen-Wechselrichter 7 ist je nach Schaltstellung der Schalter 16 ein Zwölfpulsgleichrichter oder ein Sechspulsgleichrichter. Die am Ausgang auftretende Zwischenkreis-Gleichspannung U_{Z} wird über die beiden Durchführungsfilter 8 bei Stromfluss u.a. auch von ihrer Restwelligkeit befreit. Im Wesentlichen dienen die Durchführungsfilter 8, deren Schirmwirkung der der Hochfrequenzschirmungskabine 3 entspricht, zur Unterdrückung der leitungsgebundenen elektromagnetischen Störungen in und aus der Hochfrequenzschirmungskabine 3.

Über das Kabel 9 gelangt die Zwischenkreis-Gleichspannung U_{Z} über das Eingangsfilter 11 zu den parallel geschalteten ersten Gleichspannungswandlern 12, die über die Strom-/Spannungsmesseinrichtung den Magnetisierungsstrom I_{M} liefern.

Zum Entladen (Ramp-down) des Grundfeldmagneten wird eine Stromsenke 10, die mit den Ausgängen der ersten Gleichspannungswandler 12 verbunden ist, verwendet. Eine Steuer- und Auswerteeinheit 14 steuert die ersten Gleichspannungswandler 12 und die Stromsenke 10 und wertet die Messdaten der Strom-/Spannungsmesseinrichtung 13 aus. Alternativ kann auch durch eine Netzrückspeisung mittels erster Gleichspannungswandler 12 und Dreiphasen-Wechselrichter 7 das Entladen erfolgen.

Obwohl die Erfindung im Detail durch die Ausführungsbeispiele näher illustriert und beschrieben wurde, ist die Erfindung durch die offenbarten Beispiele nicht eingeschränkt und andere Variationen können vom Fachmann daraus abgeleitet werden, ohne den Schutzumfang der Erfindung zu verlassen.

### Bezugszeichenliste

- 1: erste Schaltungseinrichtung
- 2: zweite Schaltungseinrichtung
- 3: Hochfrequenzschirmungskabine
- 4: Technikraum
- 5: Grundfeldmagnet
- 6: Transformator
- 7: Dreiphasen-Wechselrichter
- 8: Durchführungsfilter
- 9: Kabel mit vier Adern
- 10: Stromsenke
- 11: Eingangsfilter
- 12: erster Gleichspannungswandler
- 13: Strom-/Spannungsmesseinrichtung
- 14: Steuer- und Auswerteeinheit
- 15: zweiter Gleichspannungswandler
- 16: Schalter
- I_{M}: Magnetisierungsstrom
- U_{L}: Netzspannung
- U_{Z}: Zwischenkreis-Gleichspannung

## Patentansprüche

1. Magnetresonanzbildgebungsanlage aufweisend eine Hochfrequenzschirmungskabine (3) und mindestens einen Grundfeldmagneten (5),
**gekennzeichnet durch:**
- eine außerhalb der Hochfrequenzschirmungskabine (3) angeordnete erste Schaltungseinrichtung (1), die ausgebildet ist, aus einer Netzspannung (U_{L}) eine Zwischenkreis-Gleichspannung (U_{Z}) zu erzeugen, und
- eine innerhalb der Hochfrequenzschirmungskabine (3) angeordnete, mit dem Grundfeldmagneten (5) verbundene zweite Schaltungseinrichtung (2), die ausgebildet ist, aus der Zwischenkreis-Gleichspannung (U_{Z}) einen Magnetisierungsstrom (I_{M}) für den Grundfeldmagneten (5) zu erzeugen.

2. Schaltungsanordnung nach Anspruch 1,
**dadurch gekennzeichnet,**
**dass** die Netzspannung (U_{L}) eine Wechselspannung ist.

3. Magnetresonanzbildgebungsanlage nach Anspruch 1 oder 2, **dadurch gekennzeichnet,**
**dass** die erste Schaltungseinrichtung (1) in Reihe geschaltet
- einen Transformator (6) und
- einen Dreiphasen-Wechselrichter (7)
aufweist.

4. Magnetresonanzbildgebungsanlage nach Anspruch 3,
**dadurch gekennzeichnet,**
**dass** der Dreiphasen-Wechselrichter (7) als Zwölfpulsgleichrichter oder Sechspulsgleichrichter ausgebildet ist.

5. Magnetresonanzbildgebungsanlage nach einem der vorhergehenden Ansprüche,
**gekennzeichnet durch:**
- eine Stromsenke (10) der zweiten Schaltungseinrichtung (2), die ausgebildet ist, den Grundfeldmagneten (5) bei Bedarf zu entladen.

6. Magnetresonanzbildgebungsanlage nach einem der vorhergehenden Ansprüche,
**dadurch gekennzeichnet,**
**dass** die zweite Schaltungseinrichtung (2) in Reihe geschaltet
- ein Eingangsfilter (11),
- mehrere parallel geschaltete erste Gleichspannungswandler (12), die eingerichtet sind, den Magnetisierungsstrom (I_{M}) zu liefern, und
- eine Strom-/Spannungsmesseinrichtung (13)
aufweist.

7. Magnetresonanzbildgebungsanlage nach Anspruch 6,
**dadurch gekennzeichnet,**
**dass** die ersten Gleichspannungswandler (12) als regelbare Stromquellen mit einer optional einstellbaren Spannungsbegrenzung ausgebildet sind.

8. Magnetresonanzbildgebungsanlage nach den Ansprüchen 5 und 6 oder nach Anspruch 7,
**gekennzeichnet durch:**
- eine Steuer- und Auswerteeinheit (14) der zweiten Schaltungseinrichtung (2), die ausgebildet ist, die Stromsenke (10), die ersten Gleichspannungswandler (12) und die Strom-/Spannungsmesseinrichtung (13) zu steuern.

9. Magnetresonanzbildgebungsanlage nach einem der vorhergehenden Ansprüche,
**gekennzeichnet durch:**
- ein die erste und die zweite Schaltungseinrichtung (1, 2) verbindendes Kabel (9) mit mindestens einer Zuleitung und mindestens einer Rückleitung.

10. Magnetresonanzbildgebungsanlage nach einem der Ansprüche 6 bis 9,
**dadurch gekennzeichnet,**
**dass** die zweite Schaltungseinrichtung (2) einen von der Zwischenkreis-Gleichspannung (U_{Z}) gespeisten zweiten Gleichspannungswandler (15) aufweist, der ausgebildet ist, elektrische und elektronische Einheiten in der Hochfrequenzschirmungskabine (3) mit Strom zu versorgen.

11. Verfahren zum Betrieb einer Magnetresonanzbildgebungsanlage gemäss der Ansprüche 6 und 10 **dadurch gekennzeichnet,**
**dass** zum Laden des Grundfeldmagneten (5) die ersten Gleichspannungswandler (12) eingeschaltet und der zweite Gleichspannungswandler (15) ausgeschaltet werden.

12. Verfahren zum Betrieb einer Magnetresonanzbildgebungsanlage nach Anspruch 11,
**dadurch gekennzeichnet,**
**dass** nach dem Aufladen des Grundfeldmagneten (5) die ersten Gleichspannungswandler (12) ausgeschaltet und der zweite Gleichspannungswandler (15) eingeschaltet werden.

## Claims

1. Magnetic resonance imaging installation comprising a radio-frequency shielding cabin (3) and at least one basic field magnet (5),
**characterized by:**
- a first circuit device (1), which is arranged outside the radio-frequency shielding cabin (3) and which is designed to generate a DC link voltage (U_{Z}) from a grid voltage (U_{L}), and
- a second circuit device (2), which is arranged within the radio-frequency shielding cabin (3) and connected to the basic field magnet (5) and which is designed to generate a magnetization current (I_{M}) for the basic field magnet (5) from the DC link voltage (U_{Z}).

2. Circuit arrangement according to Claim 1,
**characterized**
**in that** the grid voltage (U_{L}) is an AC voltage.

3. Magnetic resonance imaging installation according to Claim 1 or 2,
**characterized**
**in that** the first circuit device (1) comprises
- a transformer (6) and
- a three-phase inverter (7)
connected in series.

4. Magnetic resonance imaging installation according to Claim 3,
**characterized**
**in that** the three-phase inverter (7) is designed as a twelve-pulse rectifier or six-pulse rectifier.

5. Magnetic resonance imaging installation according to any of the preceding claims,
**characterized by:**
- a current sink (10) of the second circuit device (2), which is designed to discharge the basic field magnet (5) as necessary.

6. Magnetic resonance imaging installation according to any of the preceding claims,
**characterized**
**in that** the second circuit device (2) comprises
- an input filter (11),
- a plurality of parallel-connected first DC-DC converters (12) which are configured to supply the magnetization current (I_{M}), and
- a current/voltage measuring device (13)
connected in series.

7. Magnetic resonance imaging installation according to Claim 6,
**characterized**
**in that** the first DC-DC converters (12) are designed as regulatable current sources with an optionally adjustable voltage limiting.

8. Magnetic resonance imaging installation according to Claims 5 and 6 or according to Claim 7,
**characterized by:**
- a control and evaluation unit (14) of the second circuit device (2), which is designed to control the current sink (10), the first DC-DC converters (12) and the current/voltage measuring device (13).

9. Magnetic resonance imaging installation according to any of the preceding claims,
**characterized by:**
- a cable (9) comprising at least one feed line and at least one return line, said cable connecting the first and second circuit devices (1, 2).

10. Magnetic resonance imaging installation according to any of Claims 6 to 9,
**characterized**
**in that** the second circuit device (2) comprises a second DC-DC converter (15), which is fed by the DC link voltage (U_{Z}) and which is designed to supply electrical and electronic units in the radio-frequency shielding cabin (3) with current.

11. Method for operating a magnetic resonance imaging installation according to Claims 6 and 10,
**characterized**
**in that**, for charging the basic field magnet (5), the first DC-DC converters (12) are switched on and the second DC-DC converter (15) is switched off.

12. Method for operating a magnetic resonance imaging installation according to Claim 11,
**characterized**
**in that**, after the charging of the basic field magnet (5), the first DC-DC converters (12) are switched off and the second DC-DC converter (15) is switched on.

## Revendications

1. Installation d'imagerie par résonance magnétique comportant une cabine (3) de blindage de haute fréquence et au moins un aimant (5) de champ de base,
**caractérisée par** :
- un premier dispositif (1) de circuit, qui est disposé à l'extérieur de la cabine (3) de blindage de haute fréquence et qui est constitué pour produire une tension (U_{Z}) continue de circuit intermédiaire à partir d'une tension (U_{L}) de réseau, et
- un deuxième dispositif (2) de circuit, qui est disposé dans la cabine (3) de blindage de haute fréquence, qui est relié à l'aimant (5) de champ de base et qui est constitué pour produire un courant (I_{M}) d'aimantation de l'aimant (5) de champ de base à partir de la tension (U_{Z}) continue de circuit intermédiaire.

2. Montage suivant la revendication 1,
**caractérisé en ce que**
la tension (U_{L}) de réseau est une tension alternative.

3. Installation d'imagerie par résonance magnétique suivant la revendication 1 ou 2,
**caractérisée**
**en ce que** le premier dispositif (1) de circuit a, montés en série,
- un transformateur (6) et
- un onduleur (7) triphasé.

4. Installation d'imagerie par résonance magnétique suivant la revendication 3,
**caractérisée**
**en ce que** l'onduleur (7) triphasé est constitué sous la forme d'un redresseur à douze impulsions ou d'un redresseur à six impulsions.

5. Installation d'imagerie par résonance magnétique suivant l'une des revendications précédentes,
**caractérisée par**
- un puits (10) de courant du deuxième dispositif (2) de circuit, qui est constitué pour décharger l'aimant (5) de champ de base en cas de besoin.

6. Installation d'imagerie par résonance magnétique suivant l'une des revendications précédentes,
**caractérisée**
**en ce que** le deuxième dispositif (2) de circuit comporte, montés en série,
- un filtre (11) d'entrée,
- plusieurs premiers convertisseurs (12) de tension continue, conçus pour fournir le courant (I_{M}) d'aimantation, et
- un dispositif (13) de mesure du courant/de la tension.

7. Installation d'imagerie par résonance magnétique suivant la revendication 6,
**caractérisée**
**en ce que** les premiers convertisseurs (12) de tension continue sont constitués sous la forme de sources de courant réglables ayant une limitation de tension, éventuellement réglable.

8. Installation d'imagerie par résonance magnétique suivant les revendications 5 et 6 ou suivant la revendication 7, **caractérisée par** :
- une unité (14) de commande et d'exploitation du deuxième dispositif (2) de circuit, qui est constituée pour commander le puits (10) de courant, les premiers convertisseurs (12) de tension continue et le dispositif (13) de mesure du courant/de la tension.

9. Installation d'imagerie par résonance magnétique suivant l'une des revendications précédentes,
**caractérisée par** :
- un câble (9) reliant le premier et le deuxième dispositifs (1, 2) de circuit et ayant au moins une ligne d'arrivée et au moins une ligne de retour.

10. Installation d'imagerie par résonance magnétique suivant l'une des revendications 6 à 9,
**caractérisée**
**en ce que** le deuxième dispositif (2) de circuit a un deuxième convertisseur (15) de tension continue, qui est alimenté par la tension (U_{Z}) continue de circuit intermédiaire et qui est constitué pour alimenter en courant des unités électriques et électroniques dans la cabine (3) de blindage de haute fréquence.

11. Procédé pour faire fonctionner une installation d'imagerie par résonance magnétique suivant les revendications 6 et 10, **caractérisé**
**en ce que** pour charger l'aimant (5) de champ de base, on met en circuit les premiers convertisseurs (12) de tension continue et on met hors circuit le deuxième convertisseur (15) de tension continue.

12. Procédé pour faire fonctionner une installation d'imagerie par résonance magnétique suivant la revendication 11, **caractérisé**
**en ce qu'**après la charge de l'aimant (5) de champ de base, on met hors circuit les premiers convertisseurs (12) de tension continue et on met en circuit le deuxième convertisseur (15) de tension continue.
